(19) Europäisches Patentamt
European Patent Office
Office européen des brevets

(11) Veröffentlichungsnummer: **0 244 713**
**B1**

(12) # EUROPÄISCHE PATENTSCHRIFT

(45) Veröffentlichungstag der Patentschrift: **16.08.90**

(21) Anmeldenummer: **87106013.3**

(22) Anmeldetag: **24.04.87**

(51) Int. Cl.⁵: **C 07 D 211/90,**
C 07 D 211/84,
C 07 D 491/04, A 61 K 31/44
// (C07D491/04, 307:00,
211:00)

(54) **3-Amino-dihydropyridine, ein Verfahren zu ihrer Herstellung und ihre Verwendung in Arzneimitteln.**

(30) Priorität: **07.05.86 DE 3615404**

(43) Veröffentlichungstag der Anmeldung:
**11.11.87 Patentblatt 87/46**

(45) Bekanntmachung des Hinweises auf die Patenterteilung:
**16.08.90 Patentblatt 90/33**

(84) Benannte Vertragsstaaten:
**AT BE CH DE ES FR GB GR IT LI NL SE**

(56) Entgegenhaltungen:
**EP-A-0 071 819**

**CHEMICAL ABSTRACTS, Band 85, Nr. 7, 16. August 1976, Seite 511, Zusammenfassungsnr. 46396s, Columbus, Ohio, US; & JP - A - 75 131 972**

**PHARMAZIE HEUTE, Band 3, Nr. 104, 1983, Seiten 139-146; R. MANNHOLD et al.: "Pharmakologische Differenzierung von Calcium-Antagonisten"**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Stoltefuss, Jürgen, D.I.**
**Parkstrasse 20**
**D-5657 Haan 2 (DE)**
Erfinder: **Heiker, Fred Robert, Dr.**
**Pahlkestrasse 15**
**D-5600 Wuppertal 1 (DE)**
Erfinder: **Bechem, Martin, Dr.**
**Obere Bergergheide 4**
**D-5600 Wuppertal 1 (DE)**
Erfinder: **Gross, Rainer, Prof. Dr.**
**Platzhofstrasse 23**
**D-5600 Wuppertal 1 (DE)**
Erfinder: **Kayser, Michael, Dr.**
**Fleyerstrasse 231**
**D-5800 Hagen 1 (DE)**
Erfinder: **Schramm, Matthias, Dr.**
**Paffrather Strasse 38**
**D-5000 Köln 80 (DE)**
Erfinder: **Thomas, Günter, Dr.**
**Henselweg 5**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 244 713 B1

**Beschreibung**

Die Erfindung betrifft 3-Amino-dihydropyridine, ein Verfahren zu ihrer Herstellung und ihre Verwendung in Arzneimitteln, insbesondere in kreislaufbeeinflussenden Arzneimitteln.

In der allgemeinen, sehr breiten Beschreibung von EP—A—071 819 werden bereits Dihydropyridinderivate mit positiv inotroper Wirkung beschrieben. Diese Entgegenhaltung enthält jedoch keinen Hinweis auf die speziell ausgewählten 3-Amino-dihydropyridinverbindungen der vorliegenden Erfindung. In C.A. 85:46396s (1976) = JP—A—75 131972 werden u.a. auch 3-Amino-dihydropyridincarbonsäureester beschreiben, die an der Aminogrupe derivatisiert, z.B. acyliert sind. Auch diese substituierten 3-Aminopyridine zeigen bei gleicher Konzentration und sonst gleichem Substituentenmuster eine eindeutige negativ inotrope Wirksamkeit während die erfindungsgemäßen unsubsituierten 3-Aminodihydropyridine eine positiv inotrope Wirksamkeit zeigen.

Dieser Effekt konnte nicht erwartet werden zumal aus der Publikation Dtsch. Apoth. Ztg. 103, 139—146 (1983) zu erwarten war, daß eine Variation der Substituenten in 3- und 5-Position des Dihydropyridinringes keinen wesentlichen Einfluß auf die biologische Wirkung haben sollte.

Die Erfindung betrifft 3-Amino-dihydropyridine der allgemeinen Formel (I)

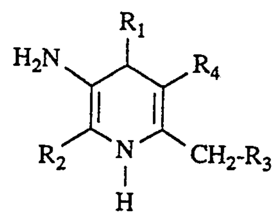

(I)

in welcher

$R_1$ für geradkettiges, verzweigtes oder cyclisches Alkyl mit bis zu 6 Kohlenstoffatomen steht, oder für Pyridyl, Thienyl, Benzoxadiazolyl oder Pyrimidyl steht, oder

für Phenyl steht, das gegenebenfalls bis zu 3-fach gleich oder verschieden substituiert ist durch $C_1$—$C_4$-Alkyl, $C_1$—$C_4$-Alkoxy, Fluor, Chlor, Brom, Carboxy, Trifluormethyl, Trifluormethoxy, Trifluormethylthio oder durch eine Gruppe der Formel

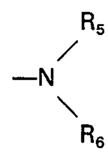

worin

$R_5$ und $R_6$ gleich oder verschieden sind und

für Wasserstoff, $C_1$—$C_4$-Alkyl, Phenyl, Benzyl, Acetyl, Benzoyl steht,

$R_2$ für geradkettiges oder verzweigtes Alkyl mit bis zu 4 Kohlenstoffatomen steht,

$R_3$ für Wasserstoff, Fluor, Chlor, Brom, Iod, Acetyloxy, Benzyloxy oder Methyl steht, und

$R_4$ für Phenyl oder

fur eine Gruppe der Formel $CO_2R_7$ steht, worin

$R_7$ für geradkettiges oder verzweigtes Alkyl mit bis zu 8 Kohlenstoffatomen steht, das gegebenenfalls durch ein Sauerstoffatom in der Kette unterbrochen ist und/oder das gegebenenfalls substituiert ist durch bis zu 7 Fluoratomen, durch Chlor, Brom, Cyano, Hydroxy, Acetyloxy, Phenyl, Phenoxy, α, β-, γ-Pyridyl oder durch eine Aminogruppe zwei gleich oder verschiedene Substituenten aus der Gruppe $C_1$—$C_2$-Alkyl oder Benzyl tragen kann oder

$R_7$ und $R_3$ gemeinsam eine Bindung darstellen, sowie deren physiologisch unbedenklichen Salze.

Besonders bevorzugt sind solche Verbindungen der allgemeinen Formel (I), in welcher

$R_1$ für geradkettiges oder verzweigtes Alkyl mit bis zu 4 Kohlenstoffatomen steht, oder

für Phenyl steht, das gegebenenfalls bis zu dreifach gleich oder verschieden substituiert ist durch $C_1$—$C_4$-Alkyl, $C_1$—$C_4$-Alkoxy, Chlor, Cyano, Trifluormethyl, Trifluormethoxy oder Trifluormethylthio,

$R_2$ für Methyl steht,

$R_3$ für Wasserstoff, Chlor, Brom, Acetyloxy, Benzyloxy oder Methyl steht, und

$R_4$ für Phenyl oder für die Gruppe der Formel —$CO_2R_7$ steht, worin

$R_7$ für geradkettiges oder verzweigtes Alkyl mit bis zu 6 Kohlenstoffatomen steht, das gegebenenfalls durch ein Sauerstoffatom in der Kette unterbrochen ist und/oder das gegebenenfalls substituiert ist durch bis zu 3 Fluor, durch Chlor, Cyano, Hydroxy, Acetyl oder N-Benzyl-N-methylamino, oder

$R_7$ und $R_3$ gemeinsam eine Bindung darstellen,

sowie deren physiologisch unbedenkliche Salze.

Physiologisch unbedenkliche Salze können Salze der freien Basen mit anorganischen oder organischen Säuren sein. Bevorzugt werden Salze mit anorganischen Säuren wie beispielsweise Salzsäure, Bromwasserstoffsäure, Phosphorsäure oder Schwefelsäure oder Salze mit organischen Carbon- oder Sulfonsäuren wie beispielsweise Essigsäure, Maleinsäure, Fumarsäure, Äpfelsäure, Citronensäure, Weinsäure, Milchsäure, Benzoesäure oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere) oder die sich nicht wie Bild und Speigelbild (Diastereomere) verhalten. Die Erfindung betrifft sowohl die Antipoden als auch die Racemformen sowie Diastereomerengemische. Die Racemformen lassen sich ebenso wie die Diastereomerengemische in bekannter Weise in die stereoisomer einheitlichen Bestandteile trennen (vgl. E. L. Eliel, Stereochemistry of Carbon Compounds, McGraw Hill, 1962).

Die erfindungsgemäßen Verbindungen der allgemeinen Formel (I) erhält man, wenn man 3-Nitro-dihydropyridine der allgemeinen Formel (II)

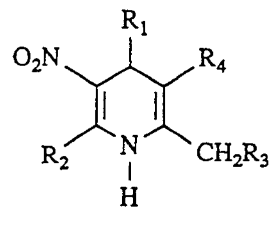

(II)

in welcher

$R_1$—$R_4$ die angegebene Bedeutung haben, in Anwesenheit eines Katalysators, in Gegenwart einer Säure und gegebenenfalls in Gegenwart eines inerten Lösungsmittels reduziert, und gegebenenfalls aus den Salzen mit Basen die freien Aminoverbindungen herstellt.

Verwendet man als Ausgangsstoff 1,4-Dihydro-2,6-dimethyl-3-nitro-4-(3-trifluormethylphenyl)pyridin-5-carbonsäuremethylester, läßt sich die Herstellung der erfindungsgemäßen Verbindungen durch folgendes Schema veranschaulichen:

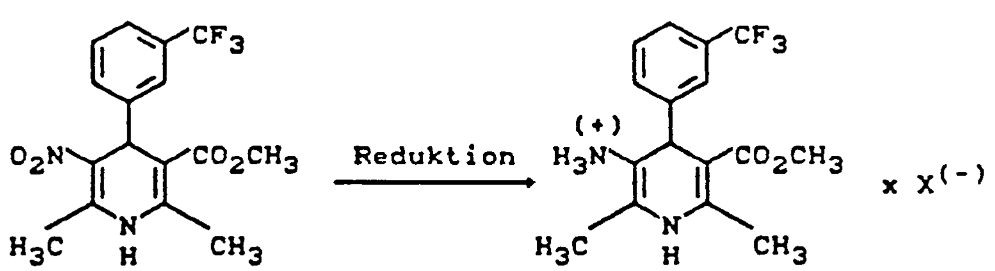


Die als Ausgangsstoffe eingesetzten 3-Nitro-Dihydropyridine sind bekannt oder können nach bekannten Methoden hergestellt werden [Belgische Patentschrift 893984).

Die Reduktion erfolgt im allgemeinen durch Hydrierung mit Metallkatalysatoren wie beispielsweise Platin, Palladium, Palladium auf Tierkohle, $PtO_2$ oder Raney-Nickel, bevorzugt mit Palladium auf Tierkohle, in Anwesenheit von Säuren.

Als Säuren können erfindungsgemäß starke Mineralsäuren aber auch organische Säuren eingesetzt werden. Bevorzugt sind dies Salzsäure, Bromwasserstoffsäure, Schwefelsäure, Phosphorsäure oder Carbonsäuren wie Essigsäure, Oxalsäure, Trifluoressigsäure oder Sulfonsäuren wie Methan-, Ethan-, Phenyl- oder Toluolsulfonsäure oder Naphthalindisulfonsäure.

Der Katalysator wird hierbei im allgemeinen in einer Menge von 0,1 bis 50 Mol-%, bevorzugt von 1 bis 10 Mol-% bezogen auf 1 mol des Nitro-dihydropyridins eingesetzt.

Die Hydrierung erfolgt im allgemeinen im Temperaturbereich von −20°C bis +100°C, bevorzugt im Bereich von 0°C bis 50°C.

Im allgemeinen erfolgt die Hydrierung mit einem Überdruck von 2 bis 200 bar, bevorzugt von 2 bis 50 bar.

Es ist ebenso möglich, die Hydrierung bei Normaldruck durchzuführen.

Als Lösungsmittel für die Hydrierung eignen sich Wasser und/oder inerte organische Lösungsmittel. Bevorzugt gehören hierzu Alkohole wie z.B. Methanol, Ethanol, Propanol oder Isopropanol, Ether wie Diethylether, Dioxan, Tetrahydrofuran, Glykolmono- oder -dimethylether, Chlorkohlenwasserstoffe wie Methylenchlorid, Chloroform oder Tetrachlorkohlenstoff, Eisessig, Trifluoressigsäure, Dimethylformamid, Essigester. Ebenso ist es möglich, Gemische der genannten Lösungsmittel einzusetzen.

Besonders bevorzugt wird die Reduktion mit Edelmetallkatalysatoren in Alkoholen in Anwesenheit von Säure bei einem Wasserstoffüberdruck durchgeführt.

Die Ausbeute der erfindungsgemäß hergestellten Verbindungen hängt ab von der Wahl des Katalysators, der Säure und den Hydrierungsbedingungen (Druck und Dauer).

Die freien Aminoverbindungen erhält man durch Umsetzen der erfindungsgemäßen Salze mit Basen. Als Basen können übliche basische Verbindungen für basische Reaktionen eingesetzt wrden. Hierzu gehören vorzugsweise Ammoniak oder Alkali- und Erdalkalihydroxide oder Carbonate wie beispielsweise Lithium-, Natrium-, Kalium-, Calcium- oder Bariumhydroxid, Natrium- oder Kaliumcarbonat, Alkalialkoholate wie beispielsweise Natriummethanolat- und -ethanolat oder Kaliummethanolat- oder -ethanolat, oder organische Basen wie beispielsweise Triethylamin, Pyridin oder 1-Methylpiperidin, Benzyltrimethylammoniumhydroxid oder Tetrabutylammoniumhydroxid.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur. Sie können deshalb in Arzneimitteln zur Beeinflussung des pathologisch veränderten Blutdrucks, als Koranartherapeutika und zur Behandlung der Herzinsuffizienz, eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herzwirkungen wurden am isoliert perfundierten Herzen von 200 g schweren Albino-Meerschweinchen beiderlei Geschlechts gefunden, das mit geeigneten Verdünnungen der Substanzen perfundiert wurde. Dazu wurden die Tiere getötet, der Thorax geöffnet, in die freipräparierte Aorta eine Metallkanüle eingebunden und der linke Vorhof eröffnet.

Das Herz wurde mit den Lungen aus dem Thorax herausgetrennt und über die Aortenkanüle an die Perfusionsapparatur bei laufender Perfusion angeschlossen. Die Lungen wurden an den Lungenwurzeln abgetrennt. Als Perfusionsmedium diente eine Krebs-Henseleit-Lösung (118 mMol/1 NNaCl, 4,8 mMol/1 KCl, 1,2 mMol/1 $MgSO_4$, 119 mMol/1 $MgSO_4$, 25 mMol/1 $NaHCO_3$, 0,013 mMol/1 NaEDTA, pH 7,4 10 mMol/1 Glukose) mit 1,2 mMol $CaCl_2$, die vor der Perfusion partikelfrei filtriert wurde. Die Herzen wurden mit konstantem Fluß mit 10 ml/min bei 32°C perfundiert. Die Herzkontraktionen wurden isovolumetrisch mit Hilfe eines in den linken Ventrikel eingeführten Latexballons gemessen und auf einem Schnellschreiber registriert.

Die Wirkungen auf die Kontraktilität einzelner Beispiele der erfindungsgemäßen Verbindungen sind in Tabelle 1 zusammengefaßt.

TABELLE 1

Bei einer Substanzkonzentration von $10^{-6}$ g/ml kommt es zu einer prozentualen Zunahme der linksvertrikulären isovolumetrischen Kontraktionsamplitude gegenüber dem gleich 100% gesetzten Ausgangswert für

| | |
|---|---|
| Bsp.-Nr. 3 | + 32% |
| Bsp.-Nr. 4 | + 33% |
| Bsp.-Nr. 5 | + 23% |
| Bsp.-Nr. 8 | + 12% |

Die neuen Wirkstoff können in bekannter Weise in die üblichen Formulierungen überführt werden, die Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Waser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.B.: Ethylalkohol, Glycerin), Trägerstoffe, wie natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulphonate und Arylsulphonate, Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten, Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und daren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Beispiel 1

3-Amino-1,4-dihydro-2,6-dimethyl-4-(3-methylphenyl)pyridin-5-carbonsäuremethylester-hydrochlorid.

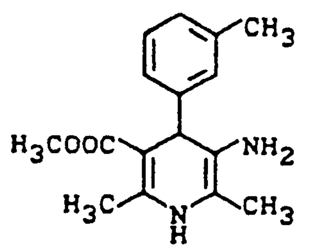

· HCl

2 g (6,63 mmol) 1,4-Dihydro-2,6-dimethyl-4-(3-methylphenyl)-3-nitro-pyridin-5-carbonsäuremethylester werden in 80 ml Methanol gelöst und mit 1,53 ml (13,2 mmol) 8,6 molarer HCl in Methanol und 200 mg 10% Palladium auf Aktivkohle versetzt und bei 3,5 bar hydriert, bis die gelbe Farbe der Lösung verschwunden ist. die Reaktion war nach 17 Minuten beendet. Es wird filtriert und eingeengt. Der ölige Eindampfrückstand wird in Acetonitril gelöst und wieder eingeengt, wobei Kristallisation eintritt. Die Kristalle werden mit Acetonitril gewaschen. Man erhält 1,3 g (63,7% der Theorie) farblose kristalle vom Schmelzpunkt 180—182°C unter Zersetzung.

Beispiel 2

3-Amino-1,4-dihydro-2,6-dimethyl-4-(3-methylphenyl)-pyridin-5-carbonsäuremethylester-semi-naphthalin-1,5-disulfonat

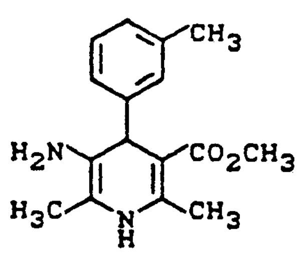

· 1/2

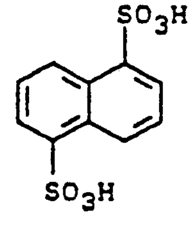

1 g (3,31 mmol) 1,4-Dihydro-2,6-dimethyl-4-(3-methylphenyl)-3-nitro-pyridin-5-carbonsäuremethylester werden in 40 ml Methanol gelöst und mit 923 mg (3,31 mmol) Naphthalin-1,5-disulfonsäure und 100 mg 10% Pd/C versetzt. Es wird unter Normaldruck hydriert, bis die Reaktion nach 1,5 Stunden beendet ist. Es wird filtriert, eingeengt, mit Aceton versetzt und wieder eingeengt. Das halbfeste Produkt wird mit Aceton und etwas Methanol verrührt, abgesaugt und mit Aceton gewaschen. Man erhält 0,5 g farblose Kristalle vom Schmelzpunkt 223°C unter Zersetzung.

Analog Beispiel 1 werden hergestellt:

Beispiel 3

3-Amino-1,4-dihydro-2,6-dimethyl-4-(3-methylphenyl)-pyridin-5-carbonsäureisopropylester-hydrochlorid vom Schmelzpunkt 187°C unter Zersetzung.

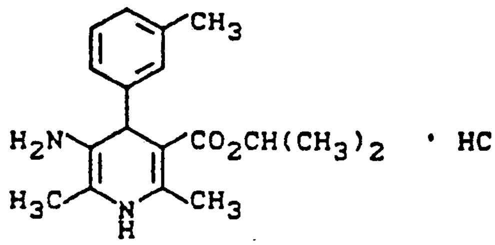

Beispiel 4

3-Amino-1,4-dihydro-2,6-dimethyl-4-(2-trifluormethylphenyl)-pyridin-5-cabronsäuremethylester-hydrochlorid vom Schmp.: 180—182°C unter Zersetzung.

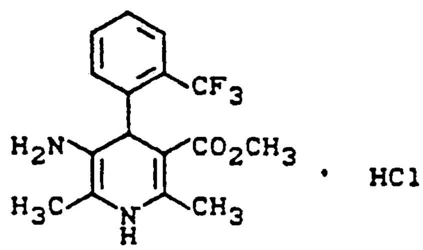

Beispiel 5

3-amino-1,4-dihydro-2,6-dimethyl-4-(2-chlorphenyl)-pyridin-5-carbonsäureisopropylester-hydrochlorid vom Schmp.: 164—166°C unter Zersetzung.

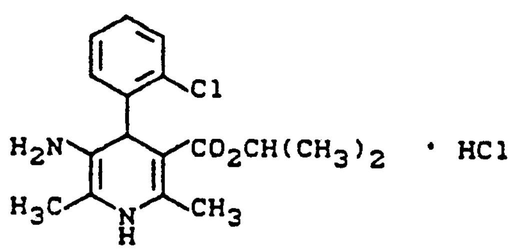

Beispiel 6

3-Amino-1,4-dihydro-2,6-dimethyl-4-isopropyl-pyridin-5-carbonsäuremethylester-hydrochlorid vom Schmp.: 159—162°C unter Zersetzung.

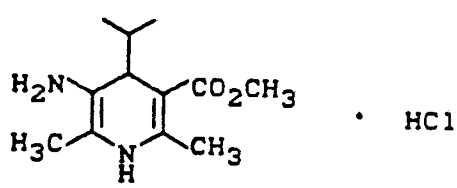

Beispiel 7

3-Amino-1,4-dihydro-2,6-dimethyl-4-(3-chlorphenyl)-pyridin-5-carbonsäure-β-hydroxyethylester-hydrochlorid

$R_f$-Wert 0,025

DC: Alurolle Kieselgel 60 F 254, Merck

Fließmittel: Toluol/Essigester im Volumenverhältnis 1:1

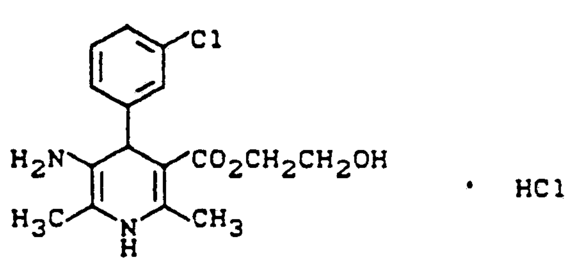

Beispiel 8

3-Amino-1,4-dihydro-2,6-dimethyl-4-(2-methylphenyl)-pyridin-5-carbonsäureisopropylester-hydrochlorid vom Schmp.: 151—56°C unter Zersetzung.

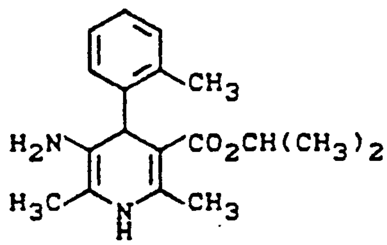

· HCl

Beispiel 9

3-Amino-1,4-dihydro-2,6-dimethyl-4-(4-trifluormethylmercaptophenyl)-5-phenyl-pyridin-hydrochlorid.

$R_f$-Wert: 0,225

DC: Alurolle Kieselgel 60 F 254, Merck

Fließmittel: Toluol/Essigester im Volumenverhältnis 1:1

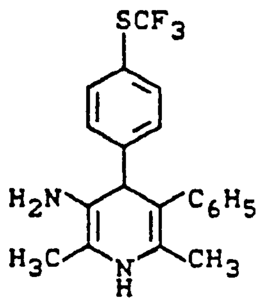

· HCl

Analog Beispiel 2 wird hergestellt:

Beispiel 10

3-Amino-2-methyl-4-(3-methylphenyl)-5-oxo-1,4,5,7-tetrahydrofuro[3,4-b]pyridin-seminaphthalin-disulfonat-(1,5) vom Schmp.: ab 260°C unter Zersetzung.

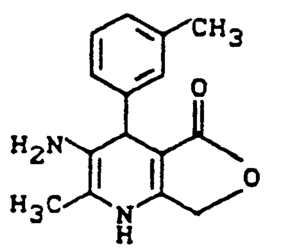

· 1/2

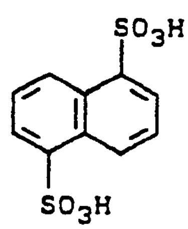

Beispiel 11

3-Amino-1,4-dihydro-2,6-dimethyl-4-(2-trifluormethylphenyl)-pyridin-5-carbonsäure-methylester

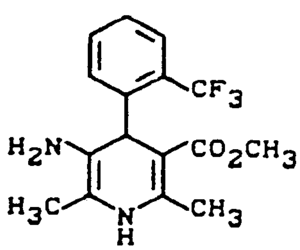

0,5 g Verbindung als Beispiel 4 werden unter Argon mit 10 ml Wasser verrührt und durch Zugabe von 5 ml Ammoniak unter Rühren alkalisch gestellt, wobei die leicht gelbe freie Base entsteht. Es wird unter Argon abgesaugt, mit Wasser gewaschen und getrocknet. Man erhält 340 mg einer leicht gelben Substanz. Schmp.: ab 70°C unter Zersetzung.

**Patentansprüche**

1. 3-Amino-dihydropyridine der allgemeinen Formel I

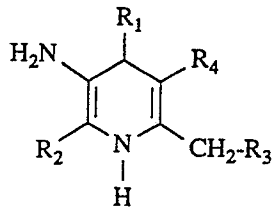

(I)

in welcher

$R_1$ für geradkettiges, verzweigtes oder cyclisches Alkyl mit bis zu 6 Kohlenstoffatomen steht, oder für Pyridyl, Thienyl, Benzoxadiazolyl oder Pyrimidyl steht, oder für Phenyl steht, das gegebenenfalls bis zu 3-fach gleich oder verschieden substituiert ist durch $C_1$—$C_4$-Alkyl, $C_1$—$C_4$-Alkoxy, Fluor, Chlor, Brom, Carboxy, Trifluormethyl, Trifluormethoxy, Trifluormethylthio oder durch eine Gruppe der Formel

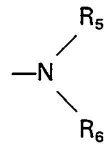

worin

$R_5$ und $R_6$ gleich oder verschieden sind und für Wasserstoff, $C_1$—$C_4$-Alkyl, Phenyl, Benzyl, Acetyl, Benzoyl steht,

$R_2$ für geradkettiges oder verzweigtes Alkyl mit bis zu 4 Kohlenstoffatomen steht,

$R_3$ für Wasserstoff, Fluor, Chlor, Brom, Iod, Acetyloxy, Benzyloxy oder Methyl steht, und

$R_4$ für Phenyl oder fur eine Gruppe der Formel $CO_2R_7$ steht, worin

$R_7$ für geradkettiges oder verzweigtes Alkyl mit bis zu 8 Kohlenstoffatomen steht, das gegebenenfalls durch ein Sauerstoffatom in der Kette unterbrochen ist und/oder das gegebenenfalls substituiert ist durch bis zu 7 Fluoratomen, durch Chlor, Brom, Cyano, Hydroxy, Acetyloxy, Phenyl, Phenoxy, α, β-, γ-Pyridyl oder durch eine Aminogruppe zwei gleich oder verschiedene Substituenten aus der Gruppe $C_1$—$C_2$-Alkyl oder Benzyl tragen kann oder

$R_7$ und $R_3$ gemeinsam eine Bindung darstellen, sowie deren physiologisch unbedenklichen Salze.

2. Verbindungen gemäß Formel I in Anspruch 1, in welchen

$R_1$ für geradkettiges, verzweigtes Alkyl mit bis zu 4 Kohlenstoffatomen steht, oder für Phenyl steht, das gegebenenfalls bis zu dreifach gleich oder verschieden substituiert ist durch $C_1$—$C_4$-Alkyl, $C_1$—$C_4$-Alkoxy, Chlor, Cyano, Trifluormethyl, Trifluormethoxy oder Trifluormethylthio,

$R_2$ für Methyl steht,

$R_3$ für Wasserstoff, Chlor, Brom, Acetyloxy, Benzyloxy oder Methyl steht, und

$R_4$ für Phenyl oder für die Gruppe der Formel —$CO_2R_7$ steht, worin

$R_7$ für geradkettiges oder verzweigtes Alkyl mit bis zu 6 Kohlenstoffatomen steht, das gegebenenfalls durch ein Sauerstoffatom in der Kette unterbrochen ist und/oder das gegebenenfalls substituiert ist durch bis zu 3 Fluor, durch Chlor, Cyano, Hydroxy, Acetyl oder N-Benzyl-N-methylamino, oder

$R_7$ und $R_3$ gemeinsam eine Bindung darstellen,

sowie deren physiologisch unbedenkliche Salze.

3. Verbindungen gemäß Ansprüchen 1 und 2 zur Bekämpfung von Erkrankungen.

4. Arzneimittel enthaltend als Wirkstoff eine Verbindung gemäß Ansprüchen 1 un 2.

5. Verfahren zur Herstellung von 3-Amino-dihydropyridinen der allgemeinen Formel I

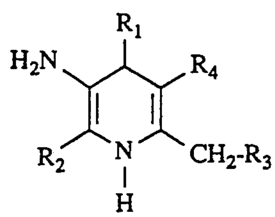

(I)

in welcher

$R_1$ für geradkettiges, verzweigtes oder cyclisches Alkyl mit bis zu 6 Kohlenstoffatomen steht, oder für Pyridyl, Thienyl, Benzoxadiazolyl oder Pyrimidyl steht, oder für Phenyl steht, das gegebenenfalls bis zu 3-fach gleich oder verschieden substituiert ist durch $C_1$—$C_4$-

Alkyl, $C_1$—$C_4$-Alkoxy, Fluor, Chlor, Brom, Carboxy, Trifluormethyl, Trifluormethoxy, Trifluormethylthio oder durch eine Gruppe der Formel

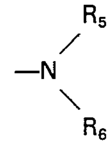

worin

$R_5$ und $R_6$ gleich oder verschieden sind und für Wasserstoff, $C_1$—$C_4$-Alkyl, Phenyl, Benzyl, Acetyl, Benzoyl steht,

$R_2$ für geradkettiges oder verzweigtes Alkyl mit bis zu 4 Kohlenstoffatomen steht,

$R_3$ für Wasserstoff, Fluor, Chlor, Brom, Iod, Acetyloxy, Benzyloxy oder Methyl steht, und

$R_4$ für Phenyl oder für eine Gruppe der Formel $CO_2R_7$ steht, worin

$R_7$ für geradkettiges oder verzweigtes Alkyl mit bis zu 8 Kohlenstoffatomen steht, das gegebenenfalls durch ein Sauerstoffatom in der Kette unterbrochen ist und/oder das gegebenenfalls substituiert ist durch bis zu 7 Fluoratomen, durch Chlor, Brom, Cyano, Hydroxy, Acetyloxy, Phenyl, Phenoxy, α, β-, γ-Pyridyl oder durch eine Aminogruppe zwei gleich oder verschiedene Substituenten aus der Gruppe $C_1$—$C_2$-Alkyl oder Benzyl tragen kann oder

$R_7$ und $R_3$ gemeinsam eine Bindung darstellen, sowie deren physiologisch unbedenklichen Salze, dadurch gekennzeichnet, daß man 3-Nitro-dihydropyridine der allgemeinen Formel II

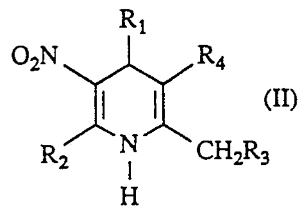 (II)

in welcher

$R_1$—$R_4$ die angegebene Bedeutung haben,

in Anwesenheit eines Katalysators, in Gegenwart einer physiologisch unbedenklichen Säure und gegebenenfalls in Gegenwart eines inerten Lösungsmittels reduziert, und gegebenenfalls aus den Salzen mit Basen die freien Aminoverbindungen herstellt.

6. Verfahren zur Herstellung von Arzneimitteln gemäß Anspruch 4, dadurch gekennzeichnet daß man Verbindungen der allgemeinen Formel I in Anspruch 5 mit üblichen Hilfs- und/oder Trägerstoffen in bekannter Weise mischt.

7. Verwendung von Verbindungen gemäß Ansprüchen 1 und 2 bei der Herstellung von Arzneimitteln zur Bekämpfung von Erkrankungen.

**Revendications**

1. 3-aminodihydropyridines de formule générale I

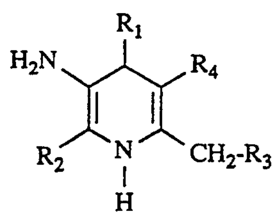 (I)

dans laquelle

$R_1$ est un groupe alkyle à chaîne droite, à chaîne ramifiée ou cyclique ayant jusqu'à 6 atomes de carbone, ou bien

un groupe pyridyle, thiényle, benzoxadiazolyle ou pyrimidyle, ou bien

un groupe phényle qui porte éventuellement jusqu'à 3 substituants, identiques ou différents, alkyle en $C_1$ à $C_4$, alkoxy en $C_1$ à $C_4$, fluoro, chloro, bromo, carboxy, trifluorométhyle, trifluorométhoxy, trifluorométhylthio ou un groupe substituant de formule

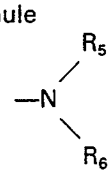

dans laquelle

$R_5$ et $R_6$ sont identiques ou différents et représentent l'hydrogène, un groupe alkyle en $C_1$ à $C_4$, phényle, benzyle, acétyle, benzoyle,

$R_2$ est un groupe alkyle à chaîne droite ou à chaîne ramifiée ayant jusqu'à 4 atomes de carbone,

$R_3$ est l'hydrogène, le fluor, le chlore, le brome, l'iode, un groupe acétyloxy, benzyloxy ou méthyle, et

$R_4$ est un groupe phényle ou un groupe de formule $CO_2R_7$, dans laquelle

$R_7$ est un radical alkyle à chaîne droite ou ramifiée ayant jusqu'à 8 atomes de carbone, dont la chaîne est interrompue le cas échéant par un atome d'oxygène et/ou qui est substitué le cas échéant par jusqu'à 7 atomes de fluor, par du chlore, du brome, un groupe cyano, hydroxy, acétyloxy, phényle, phénoxy, α-, β, γ-pyridyle ou par un groupe amino qui peut porter deux substituants identiques ou différents choisis dans le groupe des substituants alkyle en $C_1$ ou $C_2$ ou benzyle ou bien

$R_7$ et $R_3$ forment en commun une liaison ainsi que leurs sels acceptables du point de vue physiologique.

2. Composés de formule I suivant la revendication 1, dans lesquels

$R_1$ est un groupe alkyle à chaîne droite ou ramifiée ayant jusqu'à 4 atomes de carbone ou un groupe phényle qui porte le cas échéant jusqu'à 3 substituants, identiques ou différents, alkyle en $C_1$ à $C_4$, alkoxy en $C_1$ à $C_4$, chloro, cyano, trifluorométhyle, trifluorométhoxy ou trifluorométhylthio,

$R_2$ est un groupe méthyle,

$R_3$ représente l'hydrogène, le chlore, le brome, un groupe acétyloxy, benzoyloxy, ou méthyle, et

$R_4$ est un groupe phényle ou un groupe de formule $CO_2R_7$ dans laquelle

$R_7$ est un radical alkyle à chaîne droite ou ramifiée ayant jusqu'à 6 atomes de carbone, dont la chaîne est éventuellement interrompue par un atome d'oxygène et/ou qui est substitué le cas échéant par jusqu'à 3 atomes de fluor, par du chlore, un groupe cyano, hydroxy, acétyle, ou N-benzyl-N-méthylamino, ou bien $R_7$ et $R_3$ forment ensemble une liaison,

ainsi que leurs sels acceptables du point de vue physiologique.

3. Composés suivant les revendications 1 et 2, destinés à combattre des maladies.

4. Médicament contenant comme substance active un composé suivant les revendications 1 et 2.

5. Procédé de préparation de 3-aminodihydropyridines de formule générale I

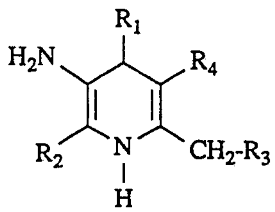

(I)

dans laquelle

$R_1$ est un groupe alkyle à chaîne droite, à chaîne ramifiée ou cyclique ayant jusqu'à 6 atomes de carbone, ou bien

un groupe pyridyle, thiényle, benzoxadiazolyle ou pyrimidyle, ou bien

un groupe phényle qui porte éventuellement jusqu'à 3 substituants, identiques ou différents, alkyle en $C_1$ à $C_4$, alkoxy en $C_1$ à $C_4$, fluoro, chloro, bromo, carboxy, trifluorométhyle, trifluorométhoxy, trifluorométhylthio ou un groupe substituant de formule

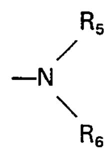

dans laquelle

$R_5$ et $R_6$ sont identiques ou différents et représentent l'hydrogène, un groupe alkyle en $C_1$ à $C_4$, phényle, benzyle, acétyle, benzoyle,

$R_2$ est un groupe alkyle à chaîne droite ou à chaîne ramifiée ayant jusqu'à 4 atomes de carbone,

$R_3$ est l'hydrogène, le flour, le chlore, le brome, l'iode, un groupe acétyloxy, benzyloxy ou méthyle, et

$R_4$ est un groupe phényle ou un groupe de formule $CO_2R_7$,

dans laquelle

$R_7$ est un radical alkyle à chaîne droite ou ramifiée ayant jusqu'à 8 atomes de carbone, dont la chaîne est interrompue le cas échéant par un atome d'oxygène et/ou qui est substitué le cas échéant par jusqu'à 7 atomes de fluor, par du chlore, du brome, un groupe cyano, hydroxy, acétyloxy, phényle, phénoxy, α-, β, γ-pyridyle ou par un groupe amino qui peut porter deux substituants identiques ou différents choisis dans le groupe des substituants alkyle en $C_1$ ou $C_2$ ou benzyle ou bien

$R_7$ et $R_3$ forment en commun une liaison

ainsi que leurs sels acceptables du point de vue physiologique, caractérisé en ce qu'on réduit des 3-nitrodihydropyridines de formule générale II

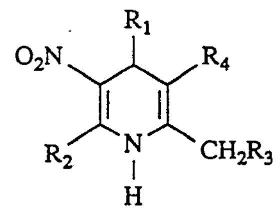

(II)

$R_1$ à $R_4$ ont la définition indiquée,

en présence d'un catalyseur, en présence d'un acide acceptable du point de vue physilogique et, le cas échéant, en présence d'un solvant inerte, et on prépare éventuellement les composés aminés libres à partir des sels, avec des bases.

6. Procédé de préparation de médicaments suivant la revendication 4, caractérisé en ce qu'on mélange d'une manière connue des composés de formule générale I suivant la revendication 5 avec des adjuvants et/ou des supports classiques.

7. Utilisation de composés suivant les revendications 1 et 2 dans la préparation de médicaments destinés à combattre des maladies.

**Claims**

1. 3-Amino-dihydropyridines of the general formula I

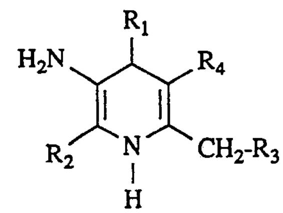

in which

$R_1$ represents straight-chain, branched or cyclic alkyl having up to 6 carbon atoms, or represents pyridyl, thienyl, benzoxadiazolyl or pyrimidyl, or represents phenyl which is optionally substituted up to 3 times, identically or differently, by $C_1$—$C_4$-alkyl, $C_1$—$C_4$-alkoxy, fluorine, chlorine, bromine, carboxyl, trifluoromethyl, trifluoromethoxy or trifluoromethylthio, or by a group of the formula in which

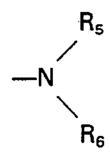

$R_5$ and $R_6$ are identical or different and represent hydrogen, $C_1$—$C_4$-alkyl, phenyl, benzyl, acetyl or benzoyl,

$R_2$ represents straight-chain or branched alkyl having up to 4 carbon atoms,

$R_3$ represents hydrogen, fluorine, chlorine, bromine, iodine, acetyloxy, benzoyloxy or methyl, and

$R_4$ represents phenyl or represents a group of the formula $CO_2R_7$, in which

$R_7$ represents straight-chain or branched alkyl which has up to 8 carbon atoms, is optionally interrupted in the chain by an oxygen atom and/or is optionally substituted by up to 7 fluorine atoms, by chlorine, bromine, cyano, hydroxyl, acetyloxy, phenyl, phenoxy, α-, β- or γ-pyridyl or by an amino group which can carry two identical or different substituents from the group comprising $C_1$—$C_2$-alkyl or benzyl, or

$R_7$ and $R_3$ together represent a bond,

and their physiologically acceptable salts.

2. Compounds according to formula I in Claim 1, in which

$R_1$ represents straight-chain or branched alkyl having up to 4 carbon atoms, or represents phenyl which is optionally substituted up to three times, identically or differently, by $C_1$—$C_4$-alkyl, $C_1$—$C_4$-alkoxy, chlorine, cyano, trifluoromethyl, trifluoromethoxy or trifluoromethylthio,

$R_2$ represents methyl,

$R_3$ represents hydrogen, chlorine, bromine, acetyloxy, benzyloxy or methyl, and

$R_4$ represents phenyl or represents the group of the formula $CO_2R_7$, in which

$R_7$ represents straight-chain or branched alkyl which has up to 6 carbon atoms, is optionally interrupted in the chain by an oxygen atom and/or is optionally substituted by up to 3 fluorine, by chlorine, cyano, hydroxyl, acetyl or N-benzyl-N-methylamino or

$R_7$ and $R_3$ together represent a bond,

and their physiologically acceptable salts.

3. Compounds according to Claims 1 and 2 for combating diseases.
4. Medicaments containing as active compound a compound according to Claims 1 and 2.
5. Process for the preparation of 3-amino-dihydropyridines of the general formula I

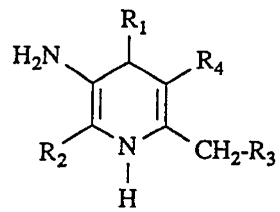

in which

$R_1$ represents straight-chain, branched or cyclic alkyl having up to 6 carbon atoms, or represents pyridyl, thienyl, benzoxadiazolyl or pyrimidyl, or represents phenyl which is optionally substituted up to 3 times, identically or differently, by $C_1$—$C_4$-alkyl, $C_1$—$C_4$-alkoxy, fluorine, chlorine, bromine, carboxyl, trifluoromethyl, trifluoromethoxy or trifluoromethylthio, or by a group of the formula

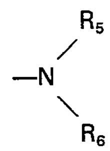

in which

$R_5$ and $R_6$ are identical or different and represent hydrogen, $C_1$—$C_4$-alkyl, phenyl, benzyl, acetyl or benzoyl,

$R_2$ represents straight-chain or branched alkyl having up to 4 carbon atoms,

$R_3$ represents hydrogen, fluorine, chlorine, bromine, iodine, acetyloxy, benzoyloxy or methyl, and

$R_4$ represents phenyl or represents a group of the formula $CO_2R_7$, in which

$R_7$ represents straight-chain or branched alkyl which has up to 8 carbon atoms, is optionally interrupted in the chain by an oxygen atom and/or is optionally substituted by up to 7 fluorine atoms, by chlorine, bromine, cyano, hydroxyl, acetyloxy, phenyl, phenoxy, α-, β- or γ-pyridyl or by an amino group which can carry two identical or different substituents from the group comprising $C_1$—$C_2$-alkyl or benzyl, or

$R_7$ and $R_3$ together represent a bond,

and their physiologically acceptable salts, characterized in that 3-nitro-dihydropyridines of the general formula II

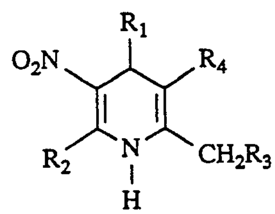

in which

$R_1$—$R_4$ have the indicated meaning, are reduced in the presence of a catalyst, in the presence of a physiologically acceptable acid, and, where appropriate, in the presence of an inert solvent, and, where appropriate, the free amino compounds are prepared from the salts with bases.

6. Process for the preparation of medicaments according to Claim 4, characterized in that compounds of the general formula I in Claim 5 are mixed in known manner with customary auxiliaries and/or vehicles.

7. Use of compounds according to Claims 1 and 2 for the preparation of medicaments for combating diseases.